# EUROPEAN PATENT APPLICATION

(11) **EP 1 982 733 A1**
(43) Date of publication of application: **22.10.2008**
(21) Application number: 07090074.1
(22) Date of filing: 17.04.2007
(51) Int. Cl.: A61K 49/10

(54) **Use of a contrast agent for magnetic resonance imaging of endoleaks**

(71) Applicant: Bayer Schering Pharma Aktiengesellschaft, 13353 Berlin (DE)
(72) Inventor: Voth, Matthias, Dr., 10115 Berlin (DE); Prokop, Matthias, 1217 KW Hilversum (NL); Cornelissen, Sandra Adriana Petronella, Dr., 1083 JP Amsterdam (NL); Mali, W.P.Th.M., 3707 EV Zeist (NL); Bartels, Lambertus Wilhelmus, Dr., 3521 XM Utrecht (NL)

(57) **Abstract**

The present invention relates to the use of an agent for the preparation of a diagnostic agent for contrast-enhanced diagnostic MR imaging of endoleak. In particular, the present invention relates to the use of a contrast agent for the visualizing of endoleak via MR imaging, e.g. after endovascular aneurysm repair. The use of an agent is disclosed, said agent comprising:
iv. an image-enhancing moiety (IEM),
v. a plasma protein binding moiety (PPBM), and
vi. a blood half-life extending moiety (BHEM),
the agent demonstrating at least about 10% binding to plasma proteins, measured in plasma, and, in a rat plasma pharmacokinetic experiment, an area under the plasma concentration versus time curve from 0 to 10 minutes, which is at least about 20% greater than observed for the combination of the IEM and the PPBM alone without the BHEM;
wherein said agent is used for the manufacture of a pharmaceutical contrast agent for diagnostic MR imaging of endoleak.

## Description

### Field of the Invention

The present invention relates to the use of a contrast agent for the preparation of a diagnostic agent for
contrast-enhanced diagnostic imaging of endoleak. In particular, the present invention relates to the use of a contrast agent for the visualizing of endoleak, e.g. after endovascular aneurysm repair.

### Background of the Invention

Patients who underwent endovascular aortic aneurysm repair (EVAR) need to be enrolled in a life-long follow-up program to monitor aneurysm size and leakage of blood (endoleak) into the aneurysm sac as the long-term efficacy of EVAR is still unknown. The follow-up program consists of arterial and delayed phase CT angiography exams (CTA) post-operatively and most cases on a regular basis (e.g. yearly) thereafter. The delayed phase is acquired 90 seconds after injection of contrast agent. Previous work showed that MRI has added value with respect to CTA in these patients.
Successfully treated abdominal aortic aneurysms are completely excluded from blood flow (no endoleak) and are expected to shrink. Shrinkage is defined as decrease in aortic diameter of more than 5 mm per year. Endoleak is an accepted etiology for aneurysms which fail to shrink. A clinical problem is formed by a large number of patients who exhibit stable or growing aneurysms without endoleak on CTA. The etiology of this phenomenon is unclear. Different causes have been proposed but were not yet visualized [White GH, J Endovasc. Ther. 2001;8:454-456] e.g. graft porosity, slow-flow endoleak and fibrinolysis. We expect that in a number of these patients, endoleak hampering shrinkage of the aneurysm is present but invisible on CTA. Endoleaks are only visualized by CTA if leakage of a detectable amount of contrast agent occurs immediately after injection. This may not be the case with slow leakage, as in this case it will take a longer time before a detectable amount of contrast agent has leaked into the aneurysm sac. By that time, the contrast agent has already disappeared from the blood. Therefore, to visualize slow leakage contrast agents are needed that stay intravascular (blood pool agent) for a prolonged period of time sufficient to enhance the MR signal of the blood. These agents allow for a longer delay between contrast fluid injection and imaging, potentially allowing the detection of slow flow endoleaks.
In Ersoy et al. (Blood Pool MR Angiography of Aortic Stent-Graft-Endoleak, AJR:182, May 2004) the blood pool MR agent Ferumoxytol (an ultrasmall superparamagnetic iron oxide core encapsulated in semisynthetic carbohydrate, Advanced Magnetics) has been used to detect endoleak. It was shown that for four patients with no endoleak detected on CT angiography, MR imaging with Ferumoxytol revealed endoleak. In one of these patients, CT angiography was of poor quality, for two patients said endoleaks were detected only on delayed MR angiograms obtained 24 hours after administration of Ferumoxytol, for the remaining patient endoleak was barely visible at the early imaging time point of 8 to 23 minutes after administration, but was more prominent on delayed MR after 24 hours. Ferumoxytol showed to be more sensitive in detecting endoleak than CT angiography, but in order to achieve this level of sensitivity an imaging time point as late as 24 hours after administration had to be chosen.
In the clinical routine an imaging time point as late as 24 hours after administration is clearly not desirable. The patient population that is routinely surveyed for the presence of endoleak is usually not hospitalized, but is outpatient. Thus the patient would have to either stay in the clinic over night or return the day after the administration. This is cost intensive and may lead to poor patient compliance.

Thus, the known diagnostic imaging methods are limited in that they cannot visualize endoleak with sufficient sensitivity in a reasonable time frame.

There is a need for diagnostic imaging agents which have improved properties with regard to visualizing endoleak. It would be useful to have diagnostic imaging agent for use in NMR / MRI that provide images of endoleak with higher sensitivity than CT. Another aspect is to provide diagnostic imaging agents for use in imaging of endoleak that allow image acquisition already at early time points. Particularly it would be useful to have diagnostic imaging agents for use in imaging endoleak at early time points which combine with an improved sensitivity compared to CT.

It was surprisingly found that an agent comprising:
i. an image-enhancing moiety (IEM),
ii. a plasma protein binding moiety (PPBM), and
iii. a blood half-life extending moiety (BHEM),
the agent demonstrating at least about 10% binding to plasma proteins and, in a rat plasma pharmacokinetic experiment, an area under the plasma concentration versus time curve from 0 to 10 minutes which is at least about 20% greater than observed for the combination of the IEM and the PPBM alone without the BHEM;
is particularly useful for the manufacture of a pharmaceutical composition for diagnostic MR imaging of endoleak.

It was also found that an agent comprising:
iv. an image-enhancing moiety (IEM),
v. a plasma protein binding moiety (PPBM), and
vi. a blood half-life extending moiety (BHEM),
the agent demonstrating at least about 10% binding to plasma proteins and, in a rat plasma pharmacokinetic experiment, an area under the plasma concentration versus time curve from 0 to 10 minutes which is at least about 20% greater than observed for the combination of the IEM and the PPBM alone without the BHEM;
is particularly useful for the manufacture of a pharmaceutical composition for diagnostic MR imaging for monitoring treated abdominal aneurysm for the presence of endoleak.

Said agents and ways of producing such agents are disclosed in WO 96/23526, the content of which is hereby incorporated by reference.

Preferred agents have a molecular weight of equal or less than 500,000 Da (500 kDa).

Such agents reveal an improved sensitivity in detecting endoleak in NMR imaging compared to CT.

Using such agents, said sensitivity can be achieved at early imaging time points, e.g. as early as 10 min after injection, preferred is an imaging time point between 10 and 120 min, more preferred between 10 and 70 min and most preferred between 30 and 70 min.

The first domain, IEM, can be any chemical or substance which is used to provide the signal or contrast in MR imaging. A particular useful IEM is a physiologically compatible metal chelate compound consisting of one or more cyclic or acyclic organic chelating agents complexed to one or more metal ions with atomic numbers 21-29, 42, 44, or 57-83.

The preferred paramagnetic metal is selected from the group of Gd(III), Fe(III), Mn(II and III), Cr(III), Cu(II), Dy(III), Tb(III), Ho(III), Er(III) and Eu(III). The most preferred is Gd(III).

Many suitable chelating ligands for use in MRI agents are known in the art and can be comprised in IEM. Preferred IEMs include DTPA, DOTA, DO3A or derivatives thereof.

The second domain, PPBM, serves to bind the agent to plasma proteins. Plasma proteins of particular interest include albumin, most preferred human serum albumin (HSA).
In order to obtain at least about 10% plasma protein binding measured in plasma, the preferred PPBM has at least 7 carbon atoms, more preferred at least 13 and most preferred at least 18 carbon atoms.

Preferred agents have at least about 10% plasma protein binding, when measured in plasma, more preferred at least about 50%, most preferred at least about 80%.

Preferred PPBM's bind to HSA. More preferred PPBM's bind to HSA with a binding affinity Ki is equal or less than 50µM when measured according to method described in US 2004/0254119 (West et al., US 10/487,025, example 3). Most preferred PPBM's bind to HSA with an affinity Ki equal or less than 15µM.

Preferred HSA-binding PPBM's have a molecular weight of less than 2,000 Da.

In the following examples of useful HSA-binding PPBM's are given:

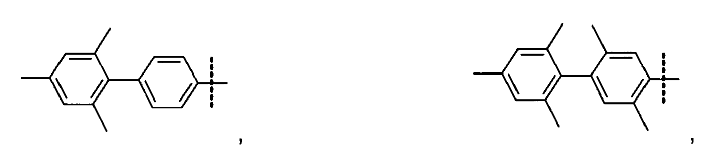

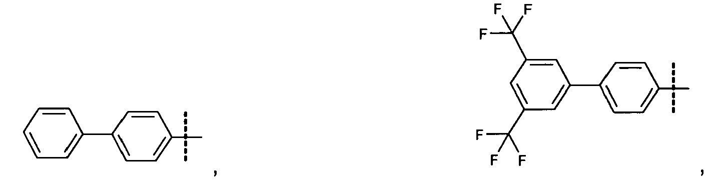

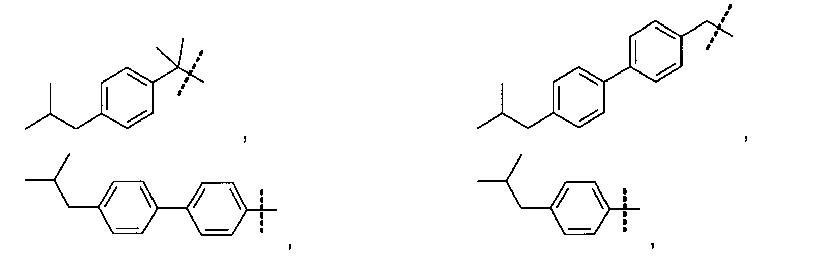

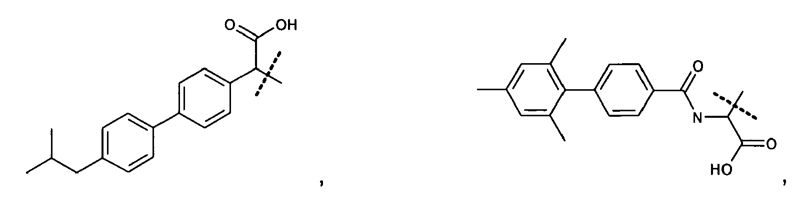

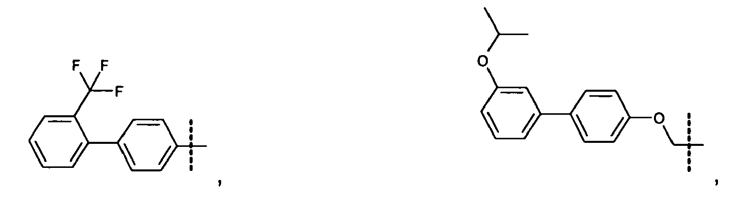

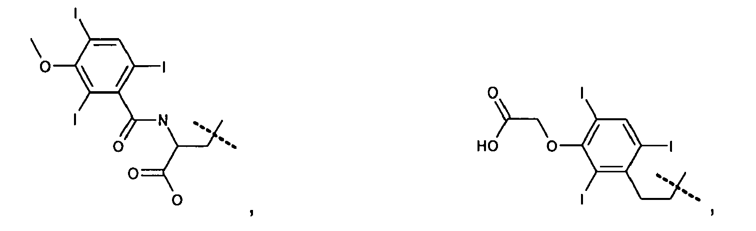

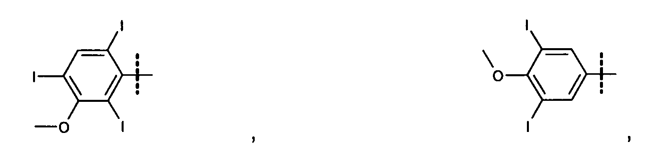

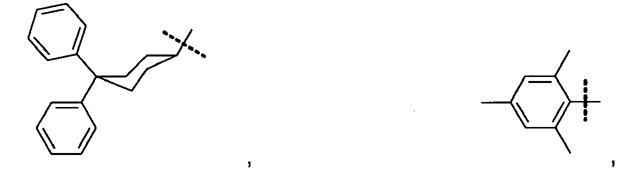

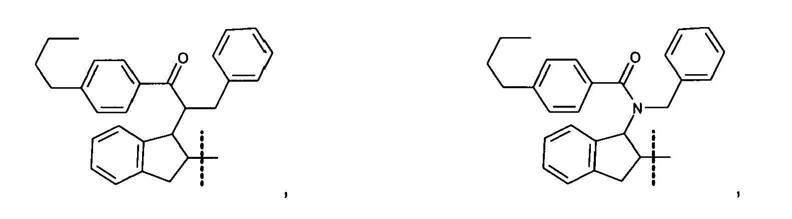

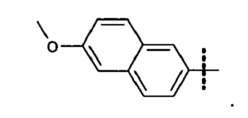

The third domain, BHEM, extends the blood half-life of the agent. Suitable BHEM's are defined in detail in WO 96/23526, from page 18 to 22, including the definition of the method to determine the area under the plasma concentration versus time curve. Preferred BHEM's result in agents with an area under the plasma concentration versus time curve from 0 to 10 minutes which is at least about 20% greater than observed for the combination of the IEM and the PPBM alone without the BHEM, when measured in the rat plasma pharmacokinetic experiment as described in WO 96/23526.

Examples of chemical groups which can serve as BHEM incluye carbon, phosphorous, tungsten, molybdenum, or sulfur atoms having attached charged or neutral heteroatoms Duch as oxygen, nitrogen, sulfur or halogens (preferred fluorine) possessing two or more lone electrón pairs (i.e., full or partial negative charge) or electropositive hydrogen atome (i.e., protonated amine) for hydrogen bonding with water. These incluye groups such as sulfone, ether, urea, thiourea, amine sulfonamide, carbamate, peptide, ester, carbonate, and acetals. Preferred groups incluye tose which posses one or more partial or full negative charges in aqueous solution at physiological pH, wherein the negatively charged atoms cannot be partially or fully neutralizad by covalent or coordinate covalent bonding to the IEM. Examples of these preferred BHEM's incluye negatively charged groups Duch as phosphate mono-ester, phosphate diester, carboxylate, and sulphonate. More preferred are tose which have phosphate groups or any ester forms thereof. Even more preferred are phosphate diesters.

The following agents are particularly preferred: 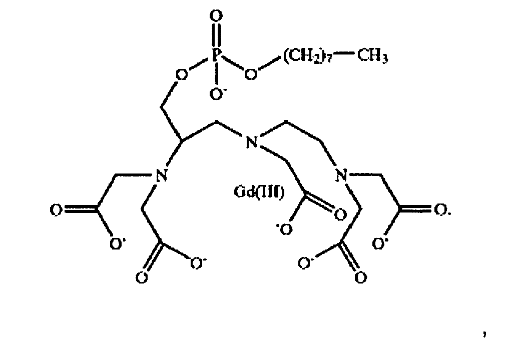 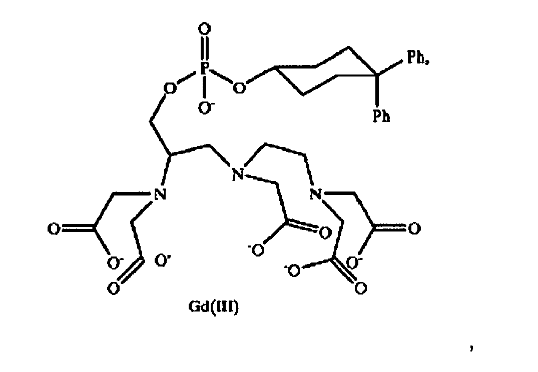 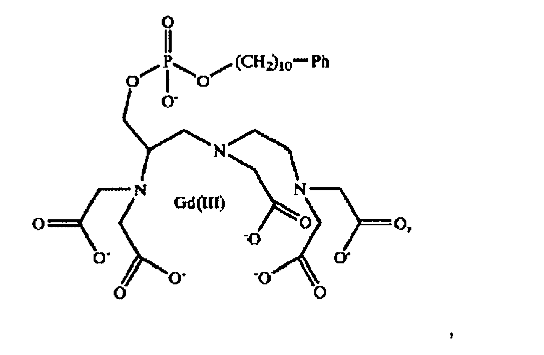 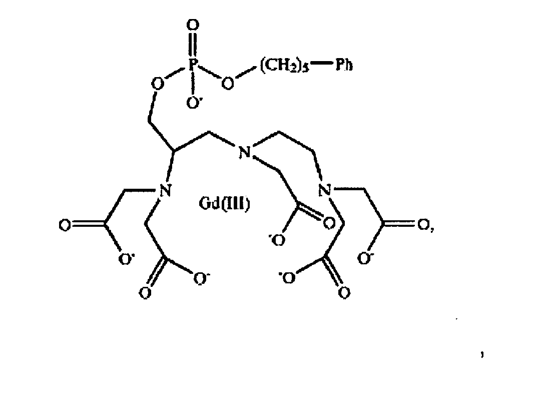 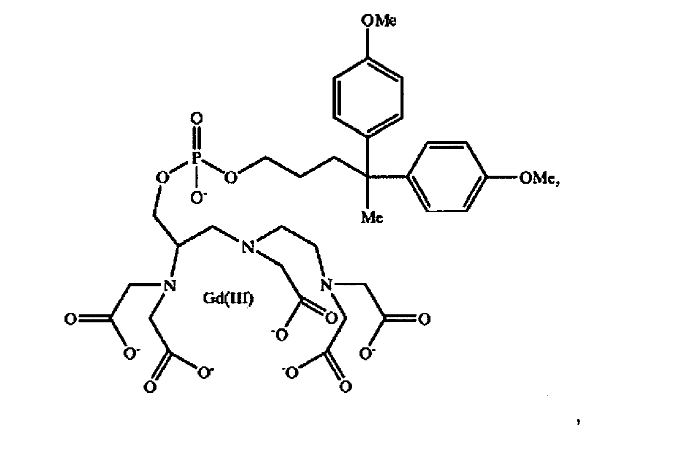 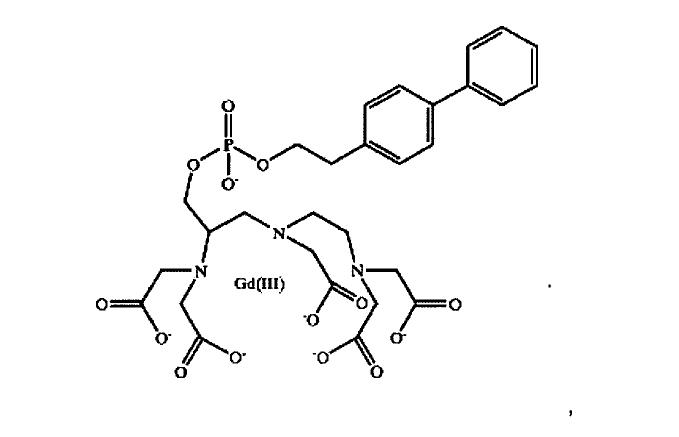 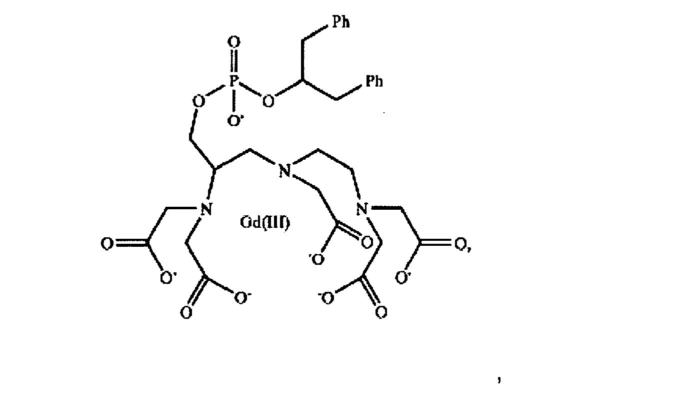 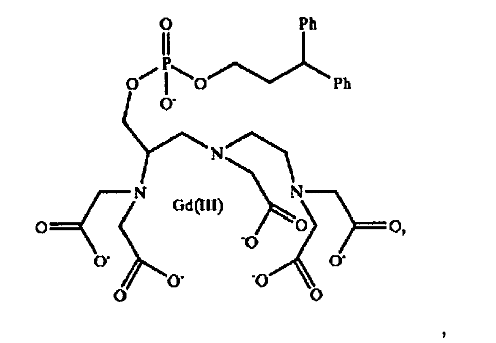 wherein Ph = phenyl and Me = methyl.

The most preferred agent according to this invention is originally disclosed in WO 96/23526 and has the following structure: 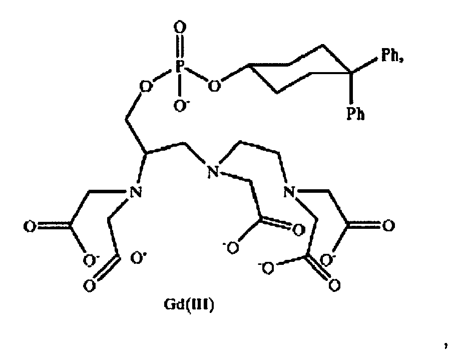 the agent is also called MS-325, Gadofosveset or Vasovist®.

The manufacture of a pharmaceutical composition for diagnostic MR imaging of endoleak is performed in a manner well known to the person skilled in the art. The respective agent, optionally with pharmaceutically acceptable additives, will be suspended in aqueous solution. The resulting solution can optionally be sterilized.

The pharmaceutical composition for diagnostic MR imaging of endoleak comprises the agent preferably in a concentration of 0,1µM to 1 M/I and will be dosed in a range of 0,0001 to 5 mM/kg.

The pharmaceutical composition for diagnostic MR imaging of endoleak can be used for enteral or parenteral administration, the preferred way of administration is parenteral, more preferred intravasal or interstitial/intracutanously, most preferred intra venously.

In order that this invention may be more fully understood, the following examples are set forth. These examples are for the purpose of illustration only and are not to be construed as limiting the scope of the invention in any way.

### Brief description of the Figures:

Figure 1 shows CT angiography image of the same patient as in Figure 2 with A) depicting the arterial phase and B) the delayed phase; no endoleak is visible.
Figure 2 shows MR images of the same patient as in Figure 1 with A) the pre-contrast image, B) the first image, and C) the second image; in C) an endoleak is clearly visible.
Figures 3 to 8 show MR images of additional patients, with A) the pre-contrast image, B) the first image, and C) the second image; in C) the arrow indicates an endoleak which is clearly visible.

### Examples:

### Visualizing endoleak after endovascular abdominal aortic aneurysm repair with the MR imaging agent Vasovist^{®}.

After successful endovascular treatment an abdominal aortic aneurysm is completely excluded from blood flow and will decrease in size. However, in a large number of patients the aneurysm does not decrease in size, but no endoleak can be visualized on CTA examinations. It is unclear why these aneurysms do not shrink. In this study we showed that by use of MRI using Vasovist® as plasma protein-binding imaging agent endoleaks can be visualized with a sensitivity that is superior to CT.

Patients who underwent endovascular aortic aneurysm repair (EVAR) need to be enrolled in a life-long follow-up program to monitor aneurysm size and leakage of blood (endoleak) into the aneurysm sac as the long-term efficacy of EVAR is still unknown. The usual follow-up program consists of arterial and delayed phase CT angiography exams (CTA) post-operatively and yearly thereafter. The delayed phase is acquired 90 seconds after injection of contrast agent. Previous work showed that MRI has added value with respect to CTA in these patients.
Successfully treated abdominal aortic aneurysms are completely excluded from blood flow (no endoleak) and are expected to shrink. Shrinkage is defined as decrease in aortic diameter of more than 5 mm per year. Endoleak is an accepted etiology for aneurysms which fail to shrink. A clinical problem is formed by a large number of patients who exhibit stable or growing aneurysms without endoleak detectable on CTA. The etiology of this phenomenon is unclear.

The purpose of this study was to visualize slow-flow endoleaks with Vasovist^{®}-enhanced MR-imaging in patients with aneurysms which do not shrink without evidence of endoleak on CTA.

### Patients and methods

12 patients were included if they had
(1) a stable or growing aneurysm, defined as increase in aneurysm diameter of more than 5 mm last year or change in aneurysm diameter of less than 5 mm last year,
(2) no evidence of endoleak on arterial and delayed phase CT angiography,
(3) a follow-up of more than 1 year, and
(4) signed the informed consent form.
Patients with MRI-incompatible endoprostheses and claustrophobia were excluded. Institutional review board approval was obtained.
Aneurysm diameters were measured by one observer on the two most recent follow-up CT angiography exams using digital calipers. These CT angiography exams were a year apart. 4 Patients had an Excluder endoprosthesis (Gore, Flagstaff, AZ, USA) 3 of which had the original Gore Excluder endoprosthesis, 1 patient had the new Excluder Low Permeability endoprosthesis, 6 patients had a Talent endoprosthesis (Medtronic Vascular), 1 patient had an EVT/Ancure (Guidant, Menlo Park, CA, USA) endoprosthesis. Median time after EVAR was 2.1 years (range 1.0 - 8.2 years). Median AAA diameter was 57 mm (range 42 - 90 mm), median time between CTA and MRA examination was 24 days (range 13 - 49 days).

### MRI contrast agent

Vasovist^{®} (gadofosveset trisodium) is a gadolinium based contrast agent which binds reversibly to human serum albumin, prolonging its intravascular time. We used Vasovist^{®} in the approved dosage of 0.03 mmol/kg. Vasovist® was administered using a Medrad infusion pump at an injection speed of 1 ml/s, followed by a 30 seconds saline chaser with the same injection speed.

### Acquisition

All MRI scans were performed on a Philips Achieva 1.5-T scanner (Philips Medical Systems, Best, The Netherlands). A wrap-around body coil was used as receive coil. Patients were scanned using a dedicated MRI-protocol, designed for follow up after EVAR.

For endoleak detection our protocol contains pre- and postcontrast T1-weighted spin echo images. Scan parameters were TR/TE/α 580 ms/15 ms/90°, slice thickness 3.0 mm, FOV 270x385 mm2, acquisition matrix 179 x 256, 60 slices. NSA=1. Acquisition time was 5 minutes 27 seconds. A regional saturation slab was placed on the abdominal fat to prevent ghosting artifacts due to breathing. Postcontrast images were acquired 3 minutes (first image) and 10 - 70 minutes after injection (second image).

### Data collection

Two observers independently evaluated the MR scans and classified them into the following categories: endoleak, possibly endoleak, and no endoleak. First and second postcontrast images were both compared to precontrast images. Leakage was defined as high signal intensity on the postcontrast image outside the lumen inside the aneurysm sac not present on the precontrast image. In case of doubt, consensus was reached after consultation of a third observer. Observers were blinded to patient's identity and other imaging studies performed.

### Results

Results of second postcontrast T1-weighted images are given, first postcontrast images in brackets (see also Table 1): Endoleak was seen in 7 (0) patients. Possible endoleak was seen in 2 (4). No endoleak was seen in 2 (7). All possible endoleaks on first postcontrast images were labeled endoleaks on the late images.

**Table 1: Endoleaks seen on first (3 min) and second (10-70min) postcontrast images for patients that were negative for endoleak measured by CT.**

| | Postcontrast phase | |
|---|---|---|
| | first | second |
| Endoleak | 0 | 7 |
| Possible leak | 4 | 2 |
| No leak | 7 | 2 |
| Nondiagnostic | 1 | 1 |
| Sum | 12 | 12 |

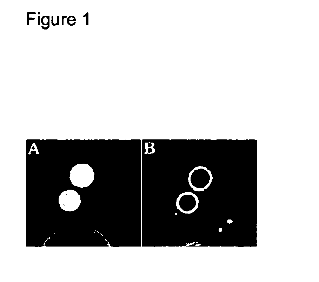

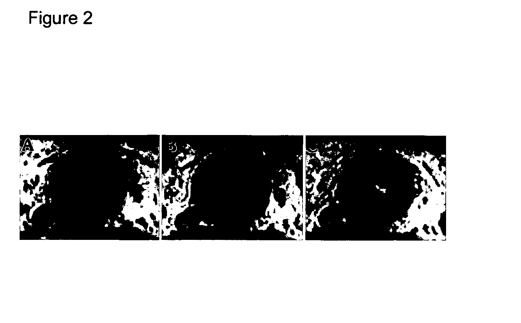

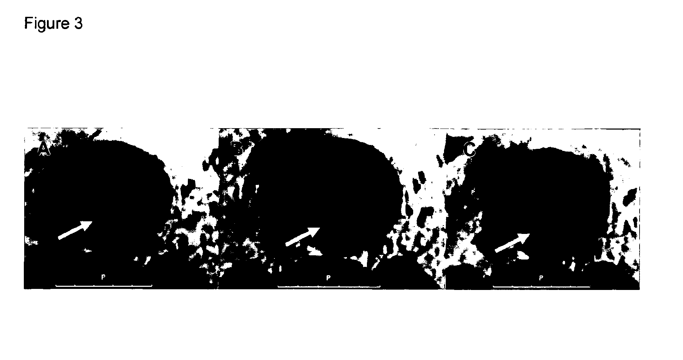

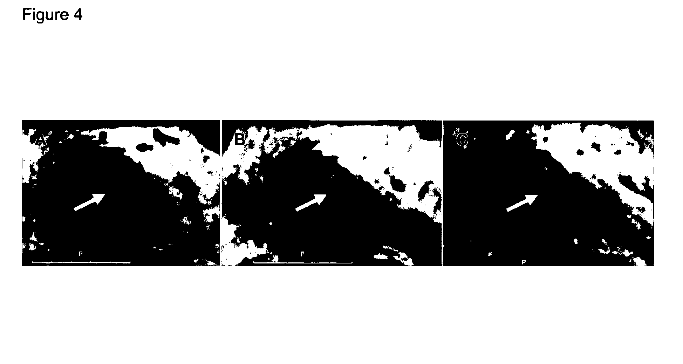

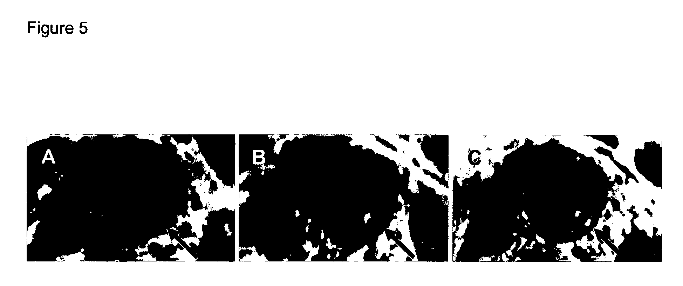

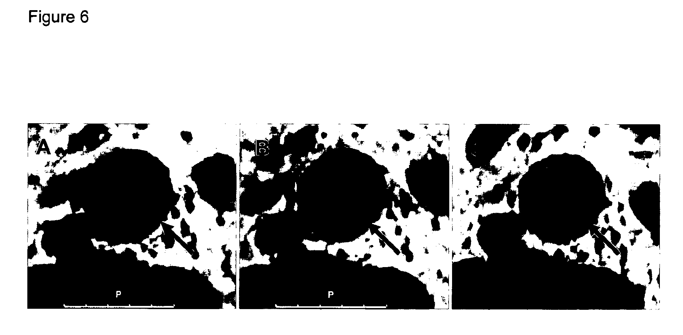

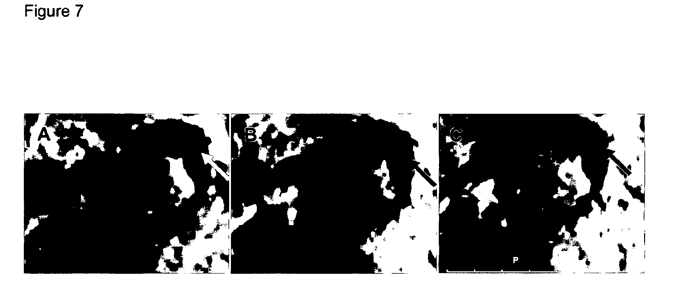

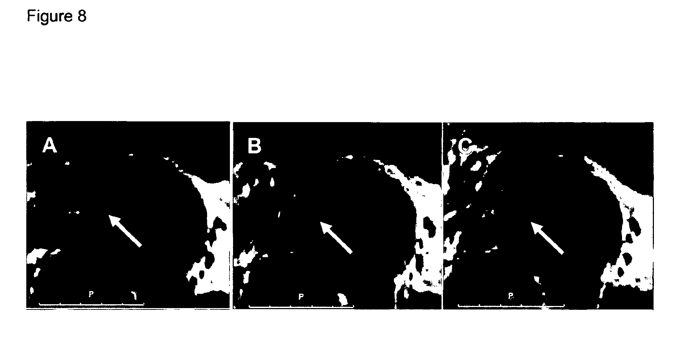

Figure 1 shows early and delayed CTA, Figure 2 shows first and second MR-images of the same patient as depicted in Figure 1, and Figures 3 to 8 show first and second MR-images of selected additional patients.

### Discussion

Our study showed that MRI with the use of the plasma protein-binding contrast agent Vasovist^{®} revealed endoleaks in more than 50% of patients who had a stable or growing aneurysm without evidence of endoleak on arterial and delayed phase CT angiography exams. Highest sensitivity for detection of endoleak was observed on the second phase images, taken within a timeframe of 10-70 min post injection.

## Claims

1. Use of an agent comprising:
i. an image-enhancing moiety (IEM),
ii. a plasma protein binding moiety (PPBM), and
iii. a blood half-life extending moiety (BHEM),
the agent demonstrating at least about 10% binding to plasma proteins, measured in plasma, and, in a rat plasma pharmacokinetic experiment, an area under the plasma concentration versus time curve from 0 to 10 minutes, which is at least about 20% greater than observed for the combination of the IEM and the PPBM alone without the BHEM;
for the manufacture of a pharmaceutical contrast agent for diagnostic MR imaging of endoleak.

2. Use of an agent according to claim 1, wherein the image-enhancing moiety is a metal chelate compound consisting of one or more cyclic or acyclic organic chelating agents complexed to one or more paramagnetic metal ions with atomic numbers 21-29, 42, 44, or 57-83.

3. Use of an agent according to one of the claims 1 to 2, wherein the agent demonstrates at least about 50% binding to plasma proteins.

4. Use of an agent according to one of the claims 1 to 3, wherein the agent demonstrates at least about 80% binding to plasma proteins.

5. Use of an agent according to one of the claims 1 to 4, wherein the agent has a molecular weight of less than 500 kDa (500.000 Da).

6. Use of an agent according to one of the claims 1 to 5, wherein the agent comprises a paramagnetic metal selected from Fe, Mn, Gd or Dy.

7. Use of an agent according to one of the claims 1 to 6, wherein the plasma protein binding moiety binds to human serum albumin.

8. Use of an agent according to one of the claims 1 to 7, wherein the PPBM is selected from the group of:

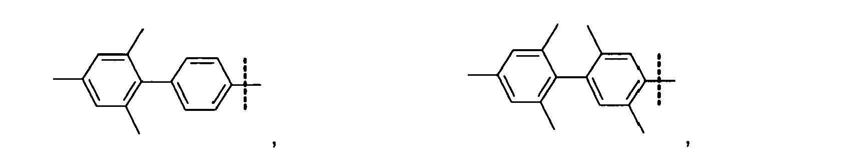

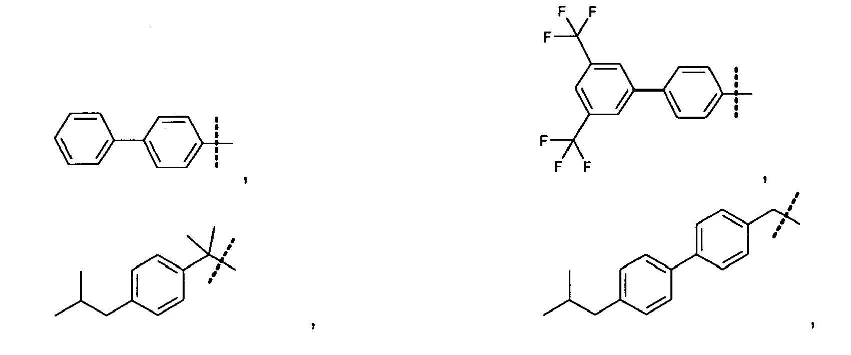

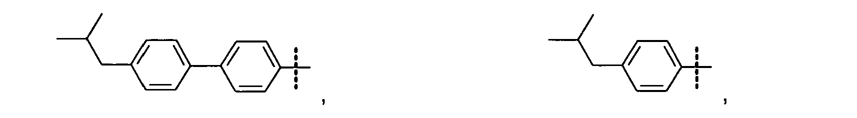

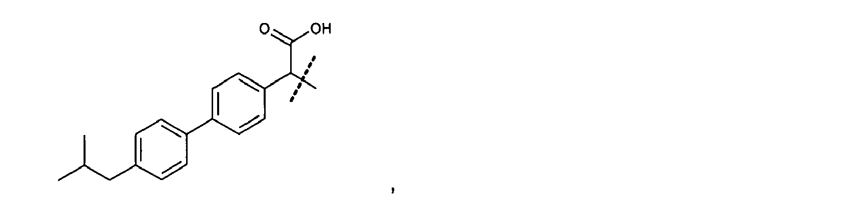

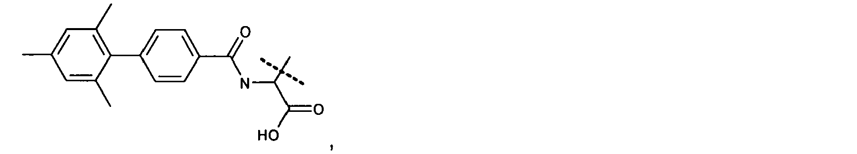

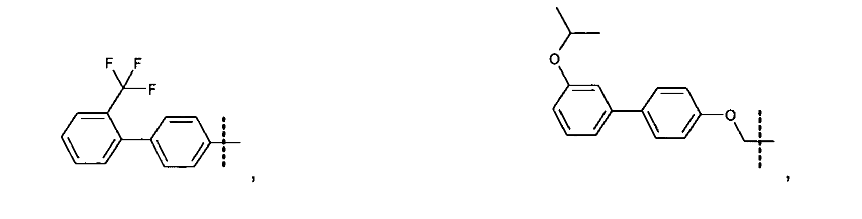

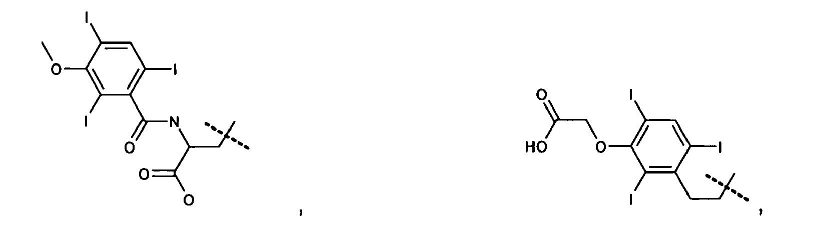

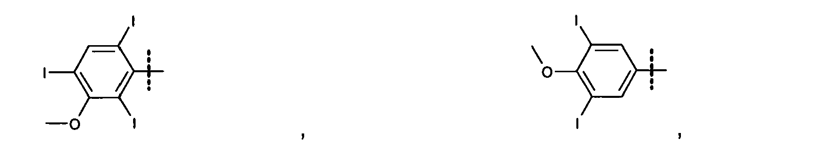

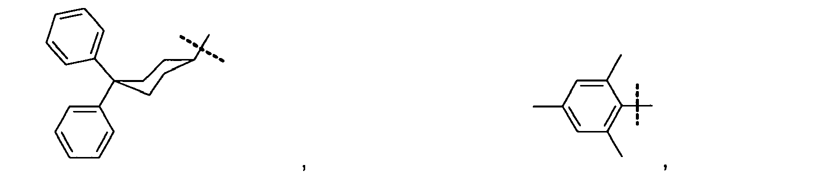

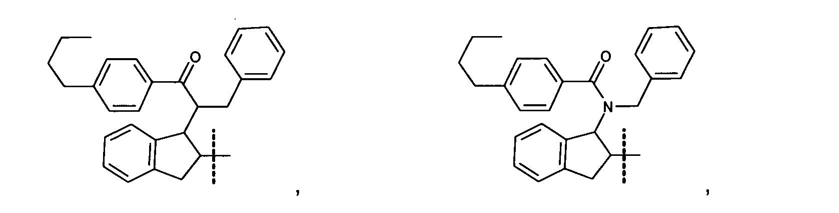

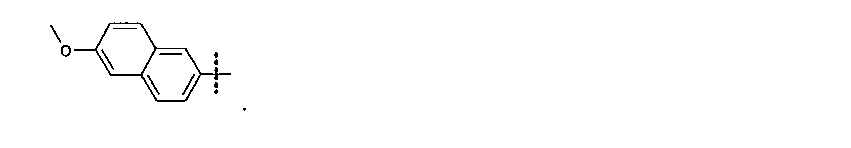

9. Use of an agent according to claim 1, wherein the agent is selected from: 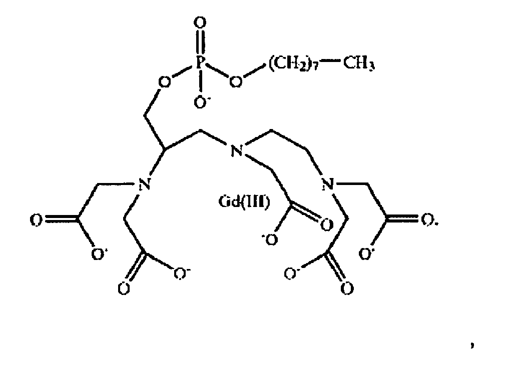 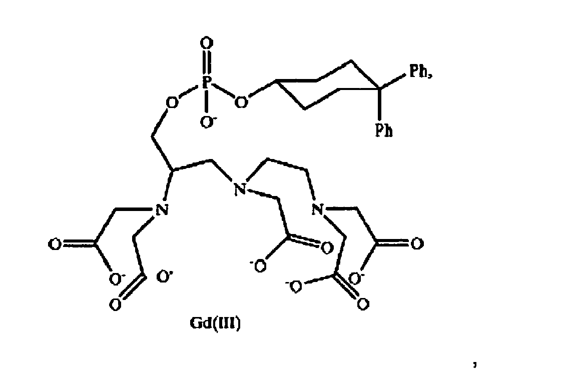 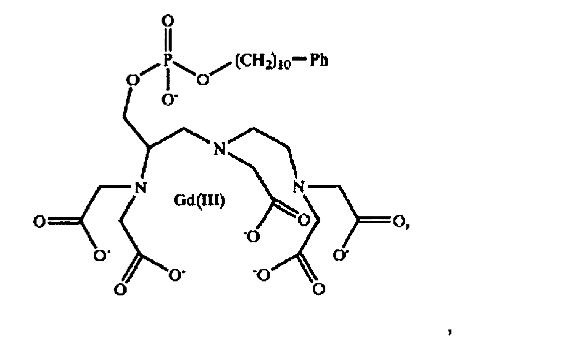 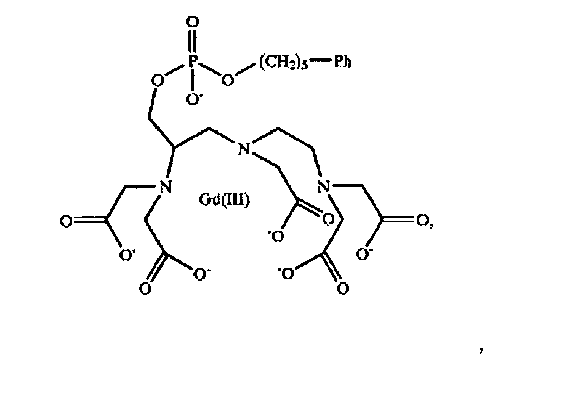 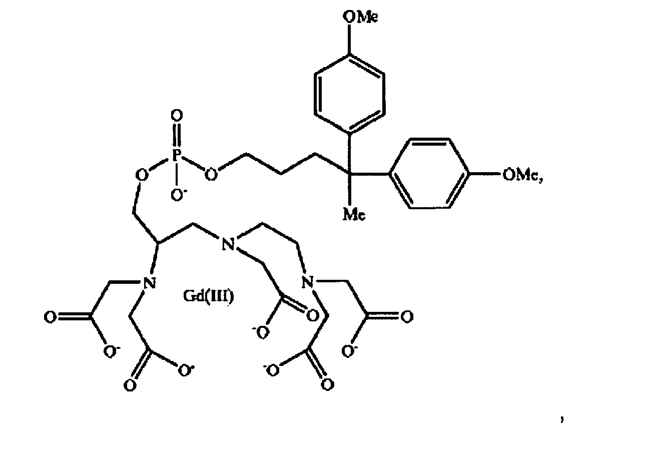 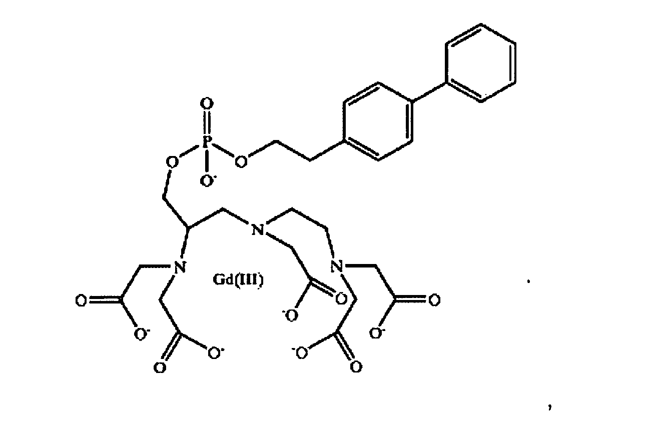 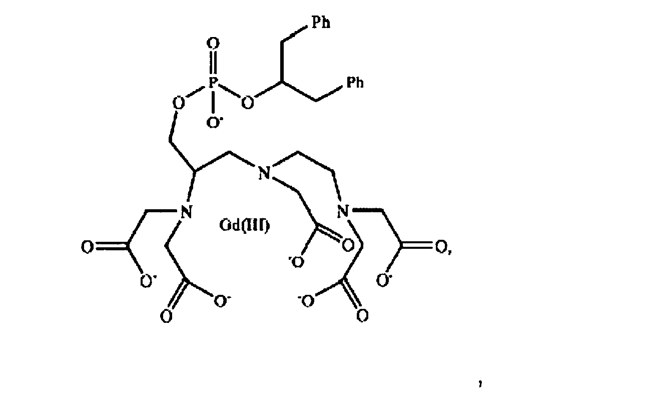 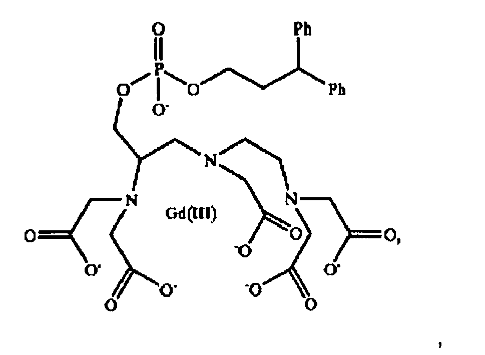 wherein Ph = phenyl and Me = methyl.

10. Use of an agent according to claim 1, wherein the agent is

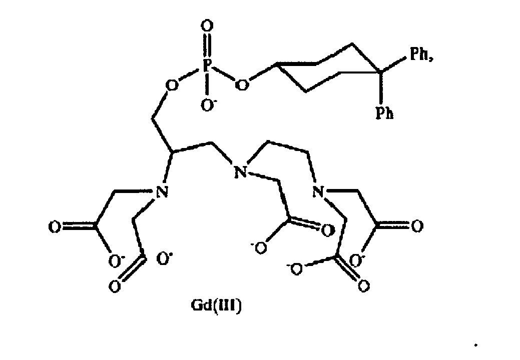

11. Use of Vasovist® for the manufacture of a pharmaceutical contrast agent for diagnostic MR imaging of endoleak.
